(19) Europäisches Patentamt
European Patent Office
Office européen des brevets

(11) Veröffentlichungsnummer: **0 124 009**
**B1**

# (12) EUROPÄISCHE PATENTSCHRIFT

(45) Veröffentlichungstag der Patentschrift: **02.11.88**

(21) Anmeldenummer: **84104328.4**

(22) Anmeldetag: **17.04.84**

(51) Int. Cl.[4]: **C 07 D 303/08**, C 07 D 303/04, C 07 D 303/22, C 07 D 301/02 // C07D249/08

## (54) Verfahren zur Herstellung von Oxiranen.

(30) Priorität: **29.04.83 DE 3315681**

(43) Veröffentlichungstag der Anmeldung: **07.11.84 Patentblatt 84/45**

(45) Bekanntmachung des Hinweises auf die Patenterteilung: **02.11.88 Patentblatt 88/44**

(84) Benannte Vertragsstaaten: **BE CH DE FR GB LI**

(56) Entgegenhaltungen:
**EP-A-0 040 345**
**EP-A-0 124 011**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Mohrmann, Karl Heinrich, Dr., Roonstrasse 61, D-5600 Wuppertal 1 (DE)**
Erfinder: **Reiser, Wolf, Dr., Kiebitzweg 12a, D-5600 Wuppertal 1 (DE)**
Erfinder: **Linke, Siegfried W., Dr. Bayer Pharma Korea Ltd., Daehan Building 10th Floor 75, Seosomondong Choong- ku Seoul (KR)**
Erfinder: **Zerbes, Rudolf, Dr., In der Beek 26, D-5600 Wuppertal 1 (DE)**

**EP 0 124 009 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein neues Verfahren zur Herstellung von bekannten Oxiranen, die als Zwischenprodukte zur Synthese von Verbindungen mit pflanzenwuchsregulierender und fungizider Wirksamkeit verwendet werden können.

Es ist bereits bekannt geworden, daß man Oxirane herstellen kann, indem man Dimethylsulfid mit Dimethylsulfat umsetzt und das dabei intermediär entstehende Trimethylsulfonium-methylsulfat anschließend mit Carbonyl-Verbindungen in Gegenwart einer starken Base und in Gegenwart eines inerten organischen Lösungsmittels, wie Acetonitril, zur Reaktion bringt (vgl. J. Amer. Chem. Soc. 87, 1353-1364 (1965) und Chem. Ber. 96, 1881-1890 (1963)).

Weiterhin ist bekannt, daß sich 2-(4-Chlorphenyl-ethyl)-2-tert.-butyl-oxiran herstellen läßt, indem man aus Dimethylsulfid und Dimethylsulfat zubereitetes Trimethylsulfonium-methylsulfat mit 1-(4-Chlorphenyl)-4,4-dimethyl-pentan-3-on in Acetonitril in Gegenwart von Natriummethylat umsetzt (vgl. EP-A-0 040 345). Die Ausbeuten bei diesem Verfahren sind gut, aber dennoch für praktische Zwecke nicht immer ausreichend. Außerdem sind dabei relativ lange Reaktionszeiten erforderlich.

Es wurde nun gefunden, daß man die bekannten Oxirane der Formel

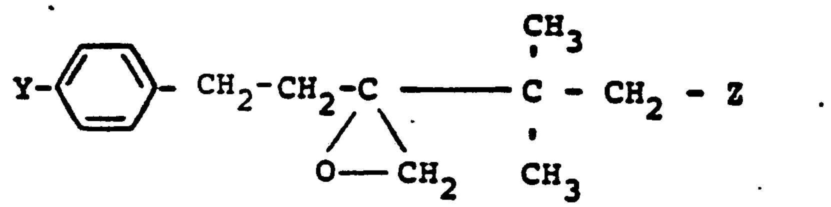

(I)

in welcher

Y für Chlor oder Phenyl steht und

Z für Wasserstoff oder Halogen steht,

erhält, wenn man Dimethylsulfid mit Dimethylsulfat in Gegenwart von tert.-Butanol behandelt und das dabei entstehende Trimethylsulfonium-methylsulfat der Formel

$(CH_3)_3S^{\oplus}\ CH_3SO_4^{\ominus}$ (II)

ohne vorherige Isolierung mit einem Keton der Formel

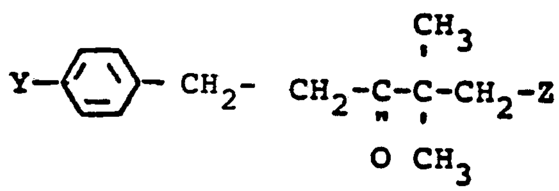

(III)

in welcher

Y und Z die oben angegebene Bedeutung haben,

in Gegenwart von Kalium-tert.-butylat sowie in Gegenwart von tert.-Butanol bei Temperaturen zwischen 0 und 60° C umsetzt.

Es ist als äußerst überraschend zu bezeichnen, daß sich Oxirane der Formel (I), z. B. das 2-(4-Chlor-phenyl-ethyl)-2-tert.-butyl-oxiran, nach dem erfindungsgemäßen Verfahren in höherer Ausbeute herstellen lassen als nach dem bisher bekannten Verfahren, bei dem Acetonitril als Lösungsmittel diente (vgl. EP-A-0 040 345). Überraschend ist auch, daß sich speziell tert.-Butanol besonders gut als Lösungsmittel eignet, während die Umsetzung in Gegenwart anderer niedrig siedender Alkohole nicht das gewünschte Ergebnis liefert.

Das erfindungsgemäße Verfahren besitzt eine Reihe von Vorteilen. So ermöglicht es die Herstellung von Oxiranen der Formel (I) in sehr guten Ausbeuten. Außerdem sind die Ausgangsstoffe relativ preisgünstig und auch in technischem Maßstab verfügbar. Darüberhinaus sind die Reaktionszeiten erheblich kürzer als bei dem bisher bekannten Verfahren zur Herstellung der Oxirane der Formel (I).

Die nach dem erfindungsgemäßen Verfahren herstellbaren Oxirane sind durch die Formel (I) definiert. In dieser Formel steht

Z steht vorzugsweise für Wasserstoff, Fluor oder Chlor.

Verwendet man bei dem erfindungsgemäßen Verfahren neben Dimethylsulfid und Dimethylsulfat das 1-(4-Chlor-phenyl)-4,4-dimethyl-pentan-3-on als Ausgangsstoff und Kalium-tert.-butylat als Base, so kann der Reaktionsablauf durch das folgende Formelschema veranschaulicht werden:

a) $(CH_3)_2S + (CH_3)_2SO_4 \longrightarrow (CH_3)_3S^{\oplus}\ CH_3SO_4^{\ominus}$

b) 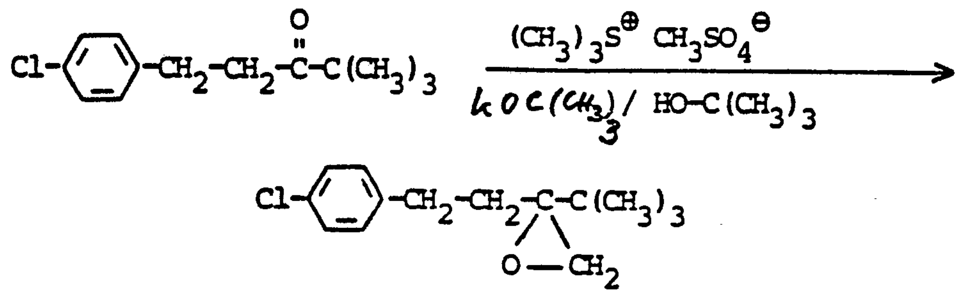


Die bei dem erfindungsgemäßen Verfahren als Ausgangsstoffe benötigten Ketone sind durch die Formel (III) definiert. In dieser Formel steht

Z steht vorzugsweise für Wasserstoff, Fluor oder Chlor.

Die Ketone der Formel (III) sind bekannt (vgl. DE-C-2 201 063, DE-A 2 705 678 und DE-A 2 737 489).

Das bei dem erfindungsgemäßen Verfahren weiterhin als Ausgangsstoff benötigte Trimethylsulfonium-methylsulfat der Formel (II) ist ebenfalls bekannt (vgl. Heterocycles 8, 397 (1977)). Es wird bei der obigen Umsetzung in frisch hergestelltem Zustand eingesetzt, indem man es durch Reaktion von Dimethylsulfid mit Dimethylsulfat erzeugt und ohne vorherige Isolierung weiterverwendet.

Die Reaktionstemperaturen können bei der Durchführung des erfindungsgemäßen Verfahrens innerhalb eines bestimmten Bereiches variiert werden. Im allgemeinen arbeitet man sowohl bei der Herstellung des Trimethylsulfonium-methylsulfats als auch bei dessen anschließender Umsetzung mit einem Keton der Formel (III) bei Temperaturen zwischen 0 und 60°C, vorzugsweise zwischen 10 und 50°C.

Das erfindungsgemäße Verfahren wird im allgemeinen bei Normaldruck durchgeführt. Es ist aber auch möglich, bei erhöhtem oder vermindertem Druck zu arbeiten.

Bei der Durchführung des erfindungsgemäßen Verfahrens werden die Mengen an Reaktionskomponenten im allgemeinen so gewählt, daß auf 1 Mol an Keton der Formel (III) im allgemeinen 1,0 bis 2,2 Mol, vorzugsweise 1,0 bis 1,5 Mol Dimethylsulfid, 1,0 bis 2,0 Mol, vorzugsweise 1,0 bis 1,5 Mol Dimethylsulfat und 1,0 bis 4,0 Mol, vorzugsweise 1,0 bis 2,0 Mol an Kalium-tert.-butylat eingesetzt werden.

Im einzelnen verfährt man bei der Durchführung des erfindungsgemäßen Verfahrens in der Weise, daß man Dimethylsulfid und Dimethylsulfat in tert.-Butanol zusammengibt, diese Lösung mehrere Stunden rührt und zu einem Gemisch aus Keton der Formel (III) und Kalium-tert.-butylat in tert.-Butanol hinzufügt. Die Aufarbeitung erfolgt nach üblichen Methoden. Im allgemeinen geht man so vor, daß man das Reaktionsgemisch mit einem Oxidationsmittel, wie zum Beispiel wäßriger Wasserstoffperoxid-Lösung oder einem Gemisch aus verdünnter, wäßriger Natrium- oder Kaliumhypochlorit-Lösung und ferner mit einem mit Wasser wenig mischbaren organischen Lösungsmittel und Wasser versetzt, die organische Phase dann abtrennt, wäscht und nach gegebenenfalls vorherigem Trocknen einengt. Das dabei anfallende Produkt kann zur weiteren Reinigung unter vermindertem Druck destilliert werden.

Die nach dem erfindungsgemäßen Verfahren herstellbaren Oxirane der Formel (I) sind wertvolle Ausgangsstoffe zur Synthese von 1-Hydroxyethyl-azol-Derivaten, welche hervorragende pflanzenwuchsregulierende und fungizide Eigenschaften besitzen (vgl. EP-A-0 040 345).

So läßt sich beispielsweise das 1-(4-Chlorphenyl)-4,4-dimethyl-3-(1,2,4-triazol-1-yl-methyl)-pentan-3-ol der Formel

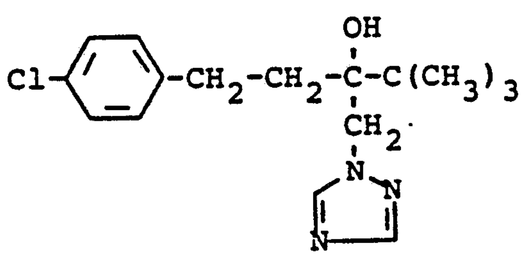

herstellen, indem man 2-(4-Chlor-phenyl-ethyl)-2-tert.-butyl-oxiran mit 1,2,4-Triazol in Gegenwart von Kaliumhydroxid umsetzt. Diese Synthese läßt sich formelmäßig wie folgt veranschaulichen:

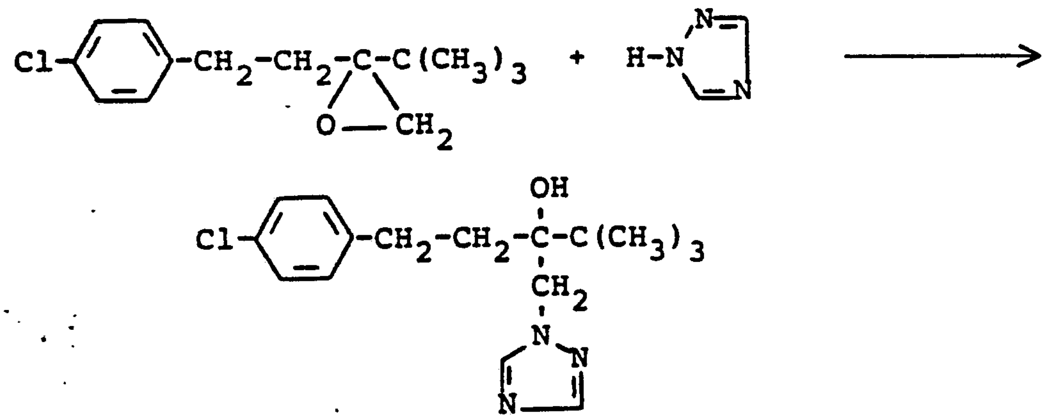

Das erfindungsgemäße Verfahren wird durch die nachfolgenden Beispiele veranschaulicht.

**Beispiel 1**

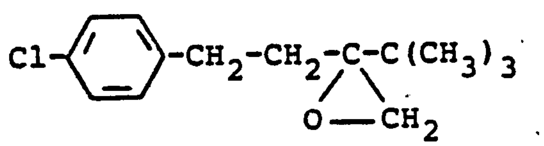

94,5 g (0,75 Mol) Dimethylsulfat wurden unter Rühren bei Raumtemperatur in ein Gemisch aus 53 g (0,85 Mol) Dimethylsulfid in 150 ml tert.-Butanol langsam eingetropft, wobei die Temperatur des Reaktionsgemisches auf 65°C anstieg. Man ließ 4 Stunden nachrühren und gab dann unter Kühlung mit einem Eisbad 117 g (0,5 Mol) eines Produktes hinzu, das zu 96 % aus 1-(4-Chlor-phenyl)-4,4-dimethyl-pentan-3-on bestand. Danach wurden 112,2 g (1 Mol) Kalium-tert.butylat schnell zugegeben, wobei die Temperatur des Reaktionsgemisches auf 40°C anstieg. Anschließend wurden die flüchtigen Anteile bis zu einer Kopftemperatur von 82°C abdestilliert. Der verbleibende Rückstand wurde nacheinander mit 2 Litern Wasser und 200 ml einer wäßrigen Natriumhypochlorit-Lösung versetzt. Danach wurden 500 ml Toluol hinzugegeben und die Phasen getrennt. Die organische Phase wurde mit Wasser neutral gewaschen, filtriert und unter vermindertem Druck eingeengt. Es verblieb ein öliger Rückstand von 113 g, der gemäß Gaschromatogramm zu 93 % aus 2-(-4-Chlor-Phenyl-ethyl)-2-tert.butyl-oxiran bestand. Danach errechnet sich eine Ausbeute von 88,1 % der Theorie.

**Vergleichsbeispiel**

Herstellung von 2-(4-Chlor-phenyl-ethyl)-2-tert.-butyl-oxiran der Formel

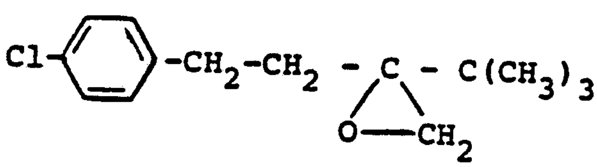

nach bekanntem Verfahren.

Zu einer Lösung von 126 ml (1,33 Mol) Dimethylsulfat in 670 ml Acetonitril wurde unter Rühren eine Lösung von 108 ml (1,47 Mol) Dimethylsulfid in 130 ml Acetonitril getropft. Man ließ das Reaktionsgemisch über Nacht stehen und versetzte dann mit 79,2 g (1,47 Mol) festem gepulvertem Natriummethylat, wobei die Temperatur des Reaktionsgemisches auf etwa 20°C gehalten wurde. Danach wurde eine Lösung von 179 g (0,8 Mol) 1-(4-Chlor-phenyl)-4,4-dimethyl-pentan-3-on in 250 ml Acetonitril zugetropft. Das Reaktionsgemisch wurde 4 Stunden nachgerührt und dann über Nacht stehen gelassen. Anschließend wurde das Reaktionsgemisch unter vermindertem Druck eingeengt, und der verbleibende Rückstand wurde in Essigester gelöst. Die dabei entstehende Lösung wurde mit Wasser gewaschen und nach dem Trocknen über Natriumsulfat durch Abziehen des Lösungsmittels unter vermindertem Druck eingeengt. Der verbleibende Rückstand wurde einer

Vakuumdestillation unterworfen. Man erhielt auf diese Weise 157 g eines Produktes, das einen Siedepunkt von 102 - 105°C bei 0,01 mbar aufwies und das gemäß Gaschromatogramm zu 53 % aus 2-(-4-Chlor-phenyl-ethyl)-2-tert.-butyloxiran bestand. Danach errechnet sich eine Ausbeute von 43 % der Theorie.

Beispiel für die Verwendung eines erfindungsgemäß herstellbaren Oxirans zur Synthese eines 1-Hydroxyethylazol-Derivates mit pflanzenwuchsregulierender und fungizider Wirksamkeit

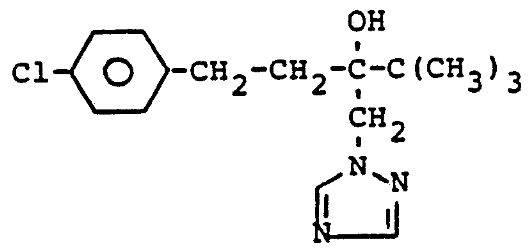

Eine Lösung von 27,1 g (0,1 Mol) eines Produktes, das zu 88 % aus 2-(4-Chlor-phenyl-ethyl)-2-tert.butyl-oxiran bestand, 8,3 g (0,12 Mol) 1,2,4-Triazol und 0,06 g (0,01 Mol) Kaliumhydroxid in 100 ml n-Propanol wurde 30 Stunden auf 95°C erhitzt. Danach ließ man abkühlen und engte das Reaktionsgemisch durch Abziehen des Lösungsmittels unter vermindertem Druck ein. Der verbliebene Rückstand wurde in Toluol aufgenommen, die dabei entstehende Suspension wurde filtriert, und das Filtrat wurde durch Abziehen des Lösungsmittels unter vermindertem Druck eingeengt. Der anfallende Rückstand wurde aus Ligroin umkristallisiert. Man erhielt auf diese Weise 30,6 g eines Produktes, das gemäß HPLC-Analyse zu 67,4 % aus 1-(4-Chlor-phenyl)-4,4-dimethyl-3-(1,2,4-triazol-1-yl-methyl)-pentan-3-ol bestand. Danach errechnet sich eine Ausbeute von 67 % der Theorie.

**Patentansprüche**

1. Verfahren zur Herstellung von Oxiranen der Formel

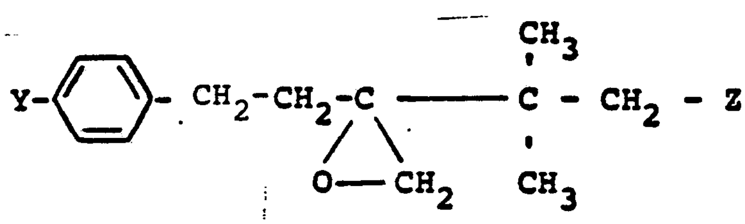
(I)

in welcher
Y für Chlor oder Phenyl steht und
Z für Wasserstoff oder Halogen steht,
dadurch gekennzeichnet, daß man Dimethylsulfid mit Dimethylsulfat in Gegenwart von tert.-Butanol behandelt und das dabei entstehende Trimethylsulfoniummethylsulfat der Formel

$(CH_3)_3S^{\oplus}\ CH_3SO_4^{\ominus}$ (II)

ohne vorherige Isolierung mit einem Keton der Formel

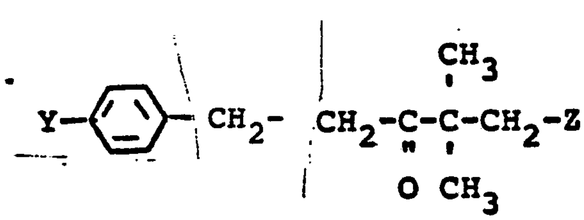
(III)

in welcher
Y und Z die oben angegebene Bedeutung haben,
in Gegenwart von Kalium-tert.-butylat sowie in Gegenwart von tert.-Butanol bei Temperaturen zwischen 0 und 60°C umsetzt.

2. Verfahren gemäß Anspruch 1, dadurch gekennzeichnet, daß man die Umsetzung bei Temperaturen zwischen 10 und 50°C durchführt.

3. Verfahren gemäß Anspruch 1, dadurch gekennzeichnet, daß man als Keton der Formel (III) 1-(4-Chlor-

phenyl)-4,4-dimethyl-pentan-3-on einsetzt.

## Claims

1. Process for the preparation of oxiranes of the formula

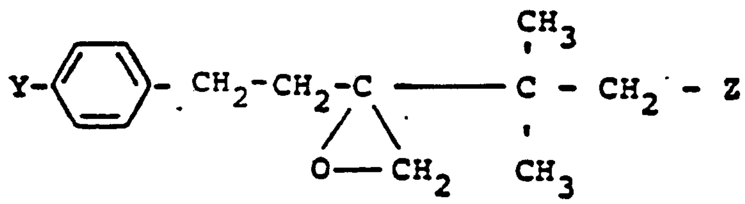

(I)

in which
Y represents chlorine or phenyl and
Z represents hydrogen or halogen,
characterized in that dimethyl sulphide is treated with dimethyl sulphate in the presence of tert.-butanol, and the trimethylsulphonium methyl sulphate produced thereby of the formula

$(CH_3)_3S^{\oplus}\ CH_3SO_4^{\ominus}$ (II)

is reacted, without previous isolation, with a ketone of the formula

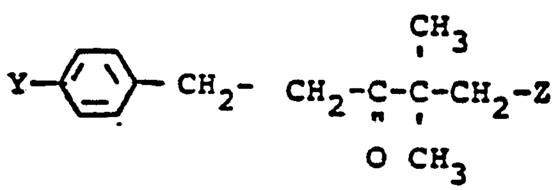

(III)

in which
Y and Z have the meanings indicated above, in the presence of potassium tert.-butylate and in the presence of tert.-butanol at temperatures between 0 and 60° C.

2. Process according to Claim 1, characterized in that the reaction is carried out at temperatures between 10 and 50° C.

3. Process according to Claim 1, characterized in that 1-(4-chlorophenyl)-4,4-dimethyl-3-pentanone is employed as the ketone of the formula (III).

## Revendications

1. Procédé de production d'oxiranes de formule

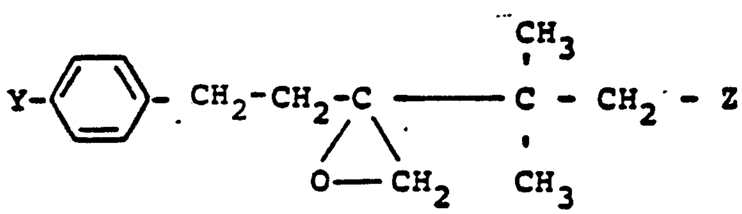

(I)

dans laquelle
Y représente le chlore ou un groupe phényle et
Z est l'hydrogène ou un halogène,
caractérisé en ce qu'on traite du sulfure de diméthyle avec du sulfate de diméthyle en présence de tertio-butanol et on fait réagir le méthylsulfate de triméthylsulfonium ainsi produit, de formule

$(CH_3)_3S^{\oplus}\ CH_3SO_4^{\ominus}$ (II)

sans isolement préalable, avec une cétone de formule

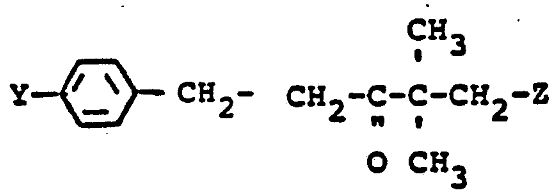

(III)

dans laquelle

Y et Z ont la définition indiquée ci-dessus, en présence de tertio-butylate de potassium ainsi qu'en présence de tertio-butanol, à des températures comprises entre 0 et 60° C.

2. Procédé suivant la revendication 1, caractérisé en ce qu'on conduit la réaction à des températures comprises entre 10 et 50° C.

3. Procédé suivant la revendication 1, caractérisé en ce qu'on utilise comme cétone de formule (III) la -(4-chloro-phényl)-4,4-diméthyl-pentane-3-one.